# FASCICULE DE BREVET EUROPEEN

(11) **EP 1 177 005 B1**
(45) Date de publication et mention de la délivrance du brevet: **29.12.2004**
(21) Numéro de dépôt: 00918964.8
(22) Date de dépôt: 13.04.2000
(51) Int. Cl.: A61M 5/50

(54) **SERINGUE A USAGE UNIQUE DITE DE SECURITE**
SICHERHEITSEINWEGSPRITZE
DISPOSABLE SAFETY SYRINGE

(30) Priorité: 14.04.1999 FR 9904630
(43) Date de publication de la demande: 06.02.2002
(73) Titulaire: D'Ussel, Bernard, 75007 Paris (FR)
(72) Inventeur: D'Ussel, Bernard, 75007 Paris (FR)
(74) Mandataire: Hammond, William
(86) Numéro de dépôt international: PCT/FR2000/000951
(87) Numéro de publication internationale: WO 2000/061210

(56) Documents cités:
- WO-A-89/03698
- WO-A-91/12038
- FR-A- 2 632 190
- US-A- 4 857 061
- US-A- 5 045 062

## Description

La présente invention est relative à une seringue à usage unique dite de sécurité.

Afin de prévenir toute contamination, il a été développé des seringues à usage unique qui doivent être jetée après utilisation : ces seringues comportent généralement un corps de pompe solidaire de l'aiguille protégée par une gaine avant emploi. Mais on s'est aperçu qu'il était possible de recharger de telles seringues et donc de les réutiliser, d'où des risques sérieux de contamination.

Comme exemple de seringue hypodermique non réutilisable, on peut citer celle décrite dans le document US-A-5.045.064. La seringue décrite comprend notamment des moyens d'obturation constitués par un plot mobile le long d'un axe fileté, un diaphragme disposé entre le plot et l'aiguille. Lors d'une injection, le liquide à injecter, poussé par le piston, entraîne un déplacement du plot par rotation le long du diaphragme qui le retient prisonnier tout en permettant au liquide de le traverser.

Aussi un des buts de la présente invention est-il de fournir une seringue à usage unique dite de sécurité, qui ne peut être remplie après une première utilisation.

Un autre but de l'invention est de fournir une telle seringue qui peut être fabriquée facilement et à moindre surcoût par rapport aux seringues jetables actuelles.

Ces buts, ainsi que d'autres qui apparaîtront par la suite, sont atteints par une seringue à usage unique dite de sécurité comprenant un corps de pompe cylindrique prolongée par une aiguille qui comprend une partie proximale munie de ladite aiguille solidaire du corps et une partie distale de plus grand diamètre comportant un piston, cette partie proximale comportant des moyens d'obturation, laquelle seringue est caractérisée par le fait que les moyens d'obturation comprennent, d'une part, un élément fixe solidaire de la paroi interne de la partie proximale et, d'autre part, un élément mobile entre l'élément fixe et le débouché de la partie distale dans la partie proximale, cet élément mobile étant constitué par un obturateur relié par une lame de ressort du type à compression dont une extrémité est solidaire de la base de l'obturateur et l'autre de la paroi interne de la partie proximale du corps de pompe.

Avantageusement, l'élément fixe est constitué par au moins une butée solidaire de la paroi interne de la partie proximale du corps de pompe.

Selon un mode de réalisation préférée de la présente invention, l'élément fixe comporte au moins deux butées régulièrement réparties sur une même cercle et solidaires de la paroi interne de la partie proximale.

Avantageusement, l'obturateur est de forme cylindro-conique, la partie conique étant dirigée vers la partie distale du corps de pompe et la partie cylindrique comportant les moyens de propulsion tels que des ailettes disposées à sa périphérie.

La description qui va suivre et qui ne présente aucun caractère limitatif, doit être lue en regard des figures annexées, parmi lesquelles :
- la figure 1 est une vue en plan d'une seringue à usage unique dite de sécurité, selon la présente invention ;
- la figure 2 est une vue en coupe selon la ligne II-II de la figure 1, avant tout usage de cette seringue ; et,
- la figure 3 une vue en coupe selon la ligne II-II de la figure 1, après remplissage de la seringue.

Ainsi qu'on peut le voir sur les figures, une seringue comporte un corps de pompe cylindrique désigné dans son ensemble par la référence 1 muni d'un piston 2 et une aiguille 3 solidaire de ce corps de pompe 1.

Le corps de pompe 1 comprend une partie proximale 4 munie de l'aiguille 3 et une partie distale 5 de plus grand diamètre comportant le piston 2 ; cette partie proximale 4 comporte des moyens d'obturation.

Ainsi que l'on comprit l'homme de métier, on désigne par « proximale » toute partie située vers l'aiguille, et par « distale » toute partie située vers le piston.

Ces moyens d'obturation comprennent, d'une part, un élément fixe 6 solidaire de la paroi interne de la partie proximale 4 et un élément mobile 7 qui peut de déplacer entre cet élément fixe 6 et le débouché 8 de la partie distale 5 dans la partie proximale 4.

L'élément fixe 6 est constitué par au moins une butée solidaire de la paroi interne de la partie proximale 4 du corps de pompe 1. Selon un mode de réalisation préféré, cet élément fixe 6 est constitué par deux butées 6a et 6b disposées symétriquement sur un même cercle sur la paroi interne de la partie proximale 4.

Quant à l'élément mobile, il est constitué, selon la présente invention, par un obturateur 7 relié par un moyen de pression 9 à la paroi interne de la partie proximale 4. Cet obturateur 7 est de forme cylindro-conique, la partie conique 7a étant dirigée vers la partie distale 5 du corps de pompe 1 et la partie cylindrique 7b comportant des moyens de propulsion tels que des ailettes 10 disposées à sa périphérie.

Ce moyen de pression est préférentiellement constitué par une lame de ressort 9 dont une extrémité est solidaire de la base de l'obturateur 7 et l'autre de la paroi interne de la partie proximale 4 du corps de pompe 1, par exemple au voisinage de la fixation de l'aiguille 3 dans celle-ci : cette lame de ressort est du type à compression.

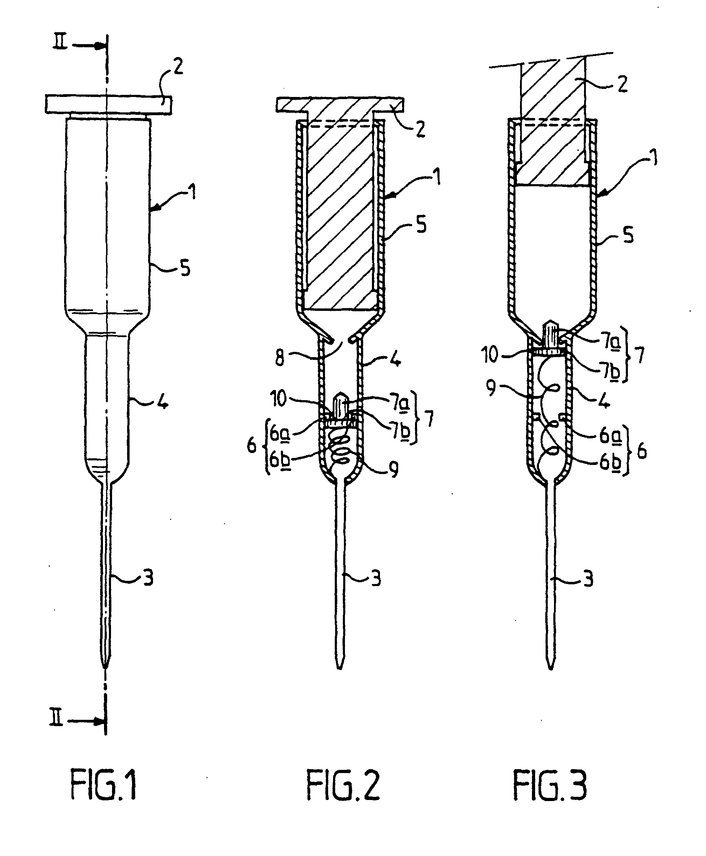

Lorsque la seringue est vide, l'obturateur 7 est maintenu en position par les ailettes 10 en coopération avec les butées 6a et 6b, comme représenté à la figure 2, la lame de ressort maintenant l'obturateur 7 appliqué contre ces butées. En actionnant le piston 2 pour remplir la seringue, le liquide aspiré pénètre dans l'aiguille 3 puis dans la partie proximale 4 sans faire décoller l'obturateur 7 des butées 6a et 6b qui forment siège, grâce notamment à la forme des ailettes 10, de telle sorte à pouvoir pénétrer dans la partie distale 5 jusqu'au niveau souhaité.

Lors de l'injection, le liquide sous la poussée du piston 2 crée un flux qui exerce sur les ailettes 10 de l'obturateur 7 une force propulsant celui-ci vers le débouché de la partie distale 5 dans la partie proximale 4, la lame de ressort 9 tendant elle aussi à ce même mouvement. La poussée du piston 2 reste cependant suffisamment forte pour contrebalancer positivement la force de propulsion exercée sur l'obturateur 7.

Lorsque l'injection est terminée, l'obturateur 7 s'applique contre le débouché de la partie distale 5 dans la partie proximale 4 sous l'effet de la lame de ressort 9 et y est maintenu appliqué : il est donc impossible d'aspirer un liquide et par suite de réutiliser une seringue selon la présente invention.

## Revendications

1. Seringue à usage unique dite de sécurité comprenant un corps de pompe cylindrique (1) prolongée par une aiguille (3) qui comprend une partie proximité (4) munie de la dite aiguille (3) solidaire dudit corps (1) et une partie distale (5) de plus grand diamètre comportant un piston (2), ladite partie proximale (4) comportant des moyens d'obturation, **caractérisée par le fait que** lesdits moyens d'obturation comprennent, d'une part, un élément fixe (6) solidaire de la paroi interne de ladite partie proximale (4) et, d'autre part, un élément mobile entre ledit élément fixe (6) et le débouché (8) de la partie distale (5) dans ladite partie proximale (4), ledit élément mobile étant constitué par un obturateur (7) relié par une lame de ressort (9) du type à compression dont une extrémité est solidaire de la base dudit obturateur (7) et l'autre de la paroi interne de ladite partie proximale (4) du corps de pompe (1).

2. Seringue selon la revendication 1, **caractérisée par le fait que** l'élément fixe (6) est constitué par au moins une butée solidaire de la paroi interne de la partie proximale (4) du corps de pompe (1).

3. Seringue selon la revendication 2, **caractérisée par le fait que** l'élément fixe comporte au moins deux butées (6a, 6b) régulièrement réparties sur une même cercle et solidaires de la paroi interne de la partie proximale (4).

4. Seringue selon la revendication 3, **caractérisée par le fait que** l'obturateur (7) est de forme cylindro-conique, la partie conique (7a) étant dirigée vers la partie distale (5) du corps de pompe (1) et la partie cylindrique (7b) comportant les moyens de propulsion tels que des ailettes (10) disposées à sa périphérie.

## Patentansprüche

1. Sicherheitseinwegspritze, umfassend einen mit einer Nadel (3) verlängerten zylindrischen Pumpkörper (1), der einen proximalen Bereich (4), der mit der fest mit dem Körper (1) verbundenen Nadel (3) versehen ist, und einen distalen Bereich (5) von größerem Durchmesser aufweist, der einen Kolben (2) beinhaltet, wobei der proximale Bereich (4) eine Verschlusseinrichtung beinhaltet, **dadurch gekennzeichnet, dass** die Verschlusseinrichtung einerseits ein unbewegliches Element (6), das fest mit der Innenwand des proximalen Bereiches (4) verbunden ist, und andererseits ein bewegliches Element zwischen dem unbeweglichen Element (6) und dem Auslass (8) des distalen Bereiches (5) im proximalen Bereich (4) umfasst, wobei das bewegliche Element von einem Verschluss (7) gebildet wird, der mit einer Bandfeder (9) vom Kompressionstyp verbunden ist, von der ein Ende fest mit der Unterseite des Verschlusses (7) und das andere Ende fest mit der Innenwand des proximalen Bereiches (4) des Pumpkörpers (1) verbunden ist.

2. Spritze nach Anspruch 1, **dadurch gekennzeichnet, dass** das unbewegliche Element (6) aus mindestens einem Anschlag besteht, der fest mit der Innenwand des proximalen Bereiches (4) des Pumpkörpers (1) verbunden ist.

3. Spritze nach Anspruch 2, **dadurch gekennzeichnet, dass** das unbewegliche Element mindest zwei Anschläge (6a, 6b) beinhaltet, die regelmäßig auf ein und demselben Kreis verteilt sind und fest mit der Innenwand des proximalen Bereiches (4) verbunden sind.

4. Spritze nach Anspruch 3, **dadurch gekennzeichnet, dass** der Verschluss (7) zylindrisch-konisch geformt ist, wobei der konische Bereich (7a) dem distalen Bereich (5) des Pumpkörpers (1) zugewandt ist und der zylindrische Bereich (7b) eine Vortriebseinrichtung, z. B. an seinem Umfang angeordnete Flügel (10), umfasst.

## Claims

1. A disposable so-called safety syringe including a cylindrical pump body (1) which is extended by a needle (3) comprises a proximal part (4) fitted with said needle (3) fastened to said body (1) and a distal part (5) of larger diameter including said plunger (2), that said proximal part (4) includes valve means, **characterised by** the fact that said valve means comprise, on the one hand, a fixed member (6) fastened to the inside wall of the proximal part (4) and, on the other part, a mobile member between said fixed member (6) and the outlet (8) from the distal part (5) into said proximal part (4), that the mobile member consists of a valve (7) connected by pressure means (9) of a compression leaf spring one end of which is fastened to the base of the valve (7) and the other to the inside wall of the proximal part (4) of the pump body (1).

2. A syringe according to claim 2, **characterised by** the fact that the fixed member (6) consists of at least one abutment fastened to the inside wall of the proximal part (4) of the pump body (1).

3. A syringe according to claim 2, **characterised by** the fact that the fixed member includes at least two abutments (6a, 6b) regularly distributed on a common circle and fastened to the inside wall of the proximal part (4).

4. A syringe according to claim 3, **characterised by** the fact that the valve (7) is of cylindrical-conical shape, the conical part (7a) being directed towards the distal part (5) of the pump body (1) and the cylindrical part (7b) including the propulsion means such as fins (10) disposed at its periphery.
